# EUROPÄISCHE PATENTSCHRIFT

(11) **EP 3 154 653 B1**
(45) Veröffentlichungstag und Bekanntmachung des Hinweises auf die Patenterteilung: **12.02.2020**
(21) Anmeldenummer: 15729785.4
(22) Anmeldetag: 09.06.2015
(51) Int. Cl.: B01D 9/00, C07C 51/43

(54) **KRISTALLISATIONSVORRICHTUNG UND VERFAHREN**
CRYSTALLISATION APPARATUS AND PROCESS
DISPOSITIF ET PROCÉDÉ DE CRISTALLISATION

(30) Priorität: 12.06.2014 DE 102014108275; 12.06.2014 US 201462011068 P
(43) Veröffentlichungstag der Anmeldung: 19.04.2017
(73) Patentinhaber: BASF SE, 67056 Ludwigshafen am Rhein (DE)
(72) Erfinder: HEILEK, Jörg, 69245 Bammental (DE); ASKANI, Rainer, 68259 Mannheim (DE); HAMMON, Ulrich, 68163 Mannheim (DE); SCHNEIDER, Wolfgang, 67098 Bad Dürkheim (DE); WALTER, Thomas, 67454 Hassloch (DE)
(74) Vertreter: BASF IP Association
(86) Internationale Anmeldenummer: PCT/EP2015/062863
(87) Internationale Veröffentlichungsnummer: WO 2015/189223

(56) Entgegenhaltungen:
- DE-A1- 4 415 844
- DE-A1- 10 332 758
- GB-A- 917 916

## Beschreibung

Die vorliegende Erfindung betrifft eine Vorrichtung und ein Verfahren zur Abtrennung eines Zielprodukts aus einer das Zielprodukt sowie Nebenprodukte enthaltenden flüssigen Phase P.

Gattungsgemäße Vorrichtungen und Verfahren zur Abtrennung eines Zielprodukts aus einer flüssigen Phase P mit Hilfe eines einen Sekundärraum und einen Primärraum aufweisenden indirekten Wärmeüberträgers sind an sich bekannt, beispielsweise aus DE 103 32 758 A1 oder DE 10 2007 004 960 A1.

Durch die Übertragung von Wärme der dem Sekundärraum zugeführten flüssigen Phase P durch die einen Sekundärraum und einen Primärraum voneinander trennende Wand (d.h. eine Wärmeübertragungsfläche) hindurch in das in dem Primärraum fließende Kühlmedium hinein, kühlt die flüssige Phase P ab, bis die Sättigungsgrenze des Zielprodukts in der flüssigen Phase P überschritten ist und einer Übersättigung durch Ausbildung von Kristallisaten des Zielprodukts entgegengewirkt wird.

Eine kristallisative Abtrennung eines Zielprodukts wird insbesondere angewendet, um dieses Zielprodukt von im Rahmen seiner Herstellung gebildeten Nebenprodukten abzutrennen. Dabei kann die Herstellung des Zielprodukts bereits unmittelbar durch chemische Reaktion in flüssiger Phase erfolgt sein. Ferner kann die Herstellung des Zielprodukts aber auch beispielsweise in der Gasphase erfolgt sein, aus der das Zielprodukt anschließend, in der Regel durch kondensative und/oder absorptive Maßnahmen, normalerweise gemeinsam mit einigen des Zielprodukt in der Gasphase begleitenden Nebenkomponenten in die flüssige Phase überführt wird.

Die kristallisative Abtrennung des Zielprodukts kann als "scharfes" thermisches Trennverfahren grundsätzlich unmittelbar aus der im Rahmen der Herstellung des Zielprodukts anfallenden, das Zielprodukt und Nebenprodukte enthaltenden flüssigen Phase P heraus erfolgen. Es hat sich jedoch als üblich herausgestellt, die vorstehend genannte flüssige Phase vorab zunächst wenigstens einem "unscharfen" thermischen Trennverfahren zum Zweck der Abtrennung einer Teilmenge der vorgenannten Nebenprodukte und Nebenkomponenten vom Zielprodukt zu unterwerfen.

Als "unscharf" wird dabei ein Trennverfahren bezeichnet, bei dem aus thermodynamischer Sicht die Zusammensetzung, die sich bei Anwendung des Trennverfahrens mit dem Zielprodukt ergibt, in thermodynamisch notwendiger Weise von der Zusammensetzung des dem Trennverfahren zu unterwerfenden Gemischs abhängig ist. Zu den im Sinne der vorliegenden Erfindung "unscharfen" thermischen Trennverfahren gehören beispielsweise die einfache Destillation, die Rektifikation, die Absorption, die fraktionierende Kondensation, die Desorption, die Extraktion, die Strippung oder die azeotrope Rektifikation.

Im Unterschied dazu ist eine kristallisative Abtrennung insofern ein "scharfes" thermisches Trennverfahren, als die Zusammensetzung der sich bildenden Kristalle aus thermodynamischer Sicht weitgehend unabhängig von der Zusammensetzung des flüssigen Ausgangsgemischs ist.

Während eine hohe Raum-Zeit-Ausbeute ein Vorteil von "unscharfen" Trennverfahren ist, ist an ihnen nachteilig, dass die mit ihnen erzielte Trennwirkung vergleichsweise beschränkt ist. Demgegenüber weisen "scharfe" Trennverfahren normalerweise vergleichsweise bescheidene Raum-Zeit-Ausbeuten auf, während sie jedoch eine sehr hohe Trennwirkung zeigen.

Ein Beispiel für ein Zielprodukt gemäß der vorliegenden Erfindung ist Acrylsäure. Acrylsäure und ihre Salze oder Ester sind ein wichtiger Grundstoff zur Herstellung von Polymerisaten für verschiedenste Anwendungsgebiete wie Klebstoffe, Superabsorber oder Bindemittel. Bei ihrer Synthese fällt die Acrylsäure üblicherweise nicht als Reinprodukt an, sondern als Teil eines Stoffgemisches, das neben der in hoher Reinheit erwünschten Zielverbindung noch unerwünschte Bestandteile wie beispielsweise Lösungsmittel, Ausgangsverbindungen und Nebenprodukte enthält. Häufig handelt es sich dabei bei dem Stoffgemisch um eine Flüssigkeit.

Acrylsäure ist beispielsweise durch katalytische Umwandlung von Glyzerin, Propan, Propen und/oder Acrolein erhältlich. Dabei werden diese Ausgangsverbindungen bevorzugt in der Gasphase, in der Regel mit inerten Gasen wie molekularem Stickstoff, CO₂ und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt. Durch kondensative und/oder absorptive Maßnahmen wird die Acrylsäure nachfolgend üblicherweise in die flüssige (kondensierte) Phase überführt. Hier findet bereits eine Grundabtrennung der Acrylsäure von den sie im Produktgasgemisch begleitenden Verbindungen statt, jedoch bei weitem nicht quantitativ.

Unter Anwendung unterschiedlichster Kombinationen von "unscharfen" und "scharfen" thermischen Trennverfahren wird die Acrylsäure schließlich in hoher Reinheit aus den vorgenannten flüssigen Phasen abgetrennt. Bestandteil solcher Verfahrenskombinationen ist vielfach das Verfahren der Suspensionskristallisation. Hierbei wird ein in seinem flüssigen Aggregatzustand befindliches, Acrylsäure enthaltendes Stoffgemisch abgekühlt, wodurch die Ausbildung von Kristallen der Acrylsäure bewirkt wird, um diese nahezu quantitativ aus dem Stoffgemisch abzutrennen.

Dabei wird die Tatsache ausgenutzt, dass beim Wachstum der aus der Acrylsäure entstehenden Kristalle die in dem flüssigen Stoffgemisch neben Acrylsäure enthaltenen Bestandteile häufig aus dem Kristallgitter verdrängt werden und in der Mutterlauge zurückbleiben.

Verfahren der Suspensionskristallisation zur kristallisativen Abtrennung von Acrylsäure sind bekannt, beispielsweise aus DE 10 2007 043 748 A1, DE 10 2007 043 758 A1 oder DE 10 2007 043 759 A1. Wie vorstehend allgemein ausgeführt, ist es anwendungstechnisch zweckmäßig, dieses Verfahren mit Hilfe eines einen Sekundärraum und wenigstens einen Primärraum aufweisenden indirekten Wärmeübertragers (Kühlers bzw. Kristallisators) durchzuführen.

Als nachteilig bei den aus dem Stand der Technik bekannten Vorrichtungen und Verfahren hat sich jedoch die Neigung zur Ausbildung von kristallisativen Verkrustungen auf der dem Sekundärraum zugewandten Seite der Trennwand herausgestellt, sowie die Fähigkeit dieser Verkrustung, auf der Trennwand haften zu bleiben. Mit der Ausbildung solcher Verkrustungen bzw. haftenden Kristallisatschichten, geht eine Minderung des Wärmedurchgangs durch die Trennwand einher. Damit wird werden pro Zeiteinheit weniger Kristalle gebildet und die Durchsatz- bzw. die Reinigungsleistung des Kristallisators geht folglich zurück.

Neben der Bildung von Verkrustungen auf den Wärmetauscherflächen des Kristallisators kommt es auch zu Verkrustungen an den Zu- und Abfuhrleitungen des Wärmeträgermediums bzw. der Produktabfuhr. Solche kristallisativen Verkrustungen können sich bei Erreichen eines bestimmten Umfangs spontan als vergleichsweise voluminöse Einzelstücke ablösen und Folgeschäden der unterschiedlichsten Art nach sich ziehen.

Beispielsweise können sie zu einer Beschädigung der zur Förderung aus dem Sekundärraum herausgeführten Kristallisatsuspension eingesetzten Pumpe führen. Zudem können durch sich lösende Einzelstücke mechanisch bewegte Teile z.B. das Wischersystem innerhalb des Kristallisators beschädigen. Daher müssen die gebildeten Kristallisatschichten auf der Trennwand und den Leitungen regelmäßig entfernt werden.

Bei der Entfernung der Kristallisatschichten wird üblicherweise die Zufuhr der zu reinigenden Flüssigkeit im Sekundärraum abgestellt und warmes Wärmeträgermedium in den Primarraum der Kühlscheiben eingeleitet. Durch die Wärmezufuhr werden die anhaftenden Kristallisatschichten abgelöst. Dabei lösen sich teilweise große Bruchstücke von Kristallisatschichten von den Trennblechen bzw. von den Zu- bzw. Abfuhrleitungen des Wärmeträgermediums und sammeln sich aufgrund der Schwerkraft auf dem Boden des Sekundärraums. Um eine Beschädigung des rotierenden Wischersystems beim Aufschmelzen in der ersten Phase des Aufschmelzvorgangs des Kristallisators durch die großen Bruchstücke der abgelösten Kristallschichten zu vermeiden, wird das Wischersystem beim Aufschmelzen in der Regel abgestellt. Da nach Abstellung des Wischersystems die zum Aufschmelzen der Kristallschichten notwendige Energie dann im wesentlichen nur durch Wärmeleitung von dem Trennblech über die Flüssigkeit übertragen wird, dauert das Aufschmelzen verhältnismäßig lange.

Angesichts der Nachteile des Standes der Technik liegt der vorliegenden Erfindung die Aufgabe zugrunde, eine Vorrichtung bereitzustellen und ein Verfahren anzugeben, mit der die abzulösenden Kristallschichten möglichst schnell aufgelöst werden und keine mechanische Beschädigung der Kristallisationseinrichtung bewirkt wird.

Die vorstehend genannte Aufgabe wird in einem ersten Aspekt der Erfindung durch eine Vorrichtung (1) gemäß Anspruch 1 gelöst.

In einem zweiten Aspekt der Erfindung wird die Aufgabe durch ein Verfahren zur Abtrennung eines Zielprodukts aus einer das Zielprodukt enthaltenden flüssigen Phase P gemäß Anspruch 4 gelöst.

Mit der erfindungsgemäßen Vorrichtung (1) und dem erfindungsgemäßen Verfahren ist es möglich, die Nachteile des Standes der Technik zu überwinden und Kristallisatschichten des Zielprodukts und Verkrustungen schonend bzw. schnell abzubauen. Insbesondere ist es mit der vorliegenden Erfindung möglich, die Stillstandzeit der erfindungsgemäßen Vorrichtung (1) gegenüber gattungsgemäßen Vorrichtungen deutlich zu verringern, so dass eine wirtschaftlichere Betriebsweise erreicht wird.

Sofern in der nachfolgenden Beschreibung im Zusammenhang mit der erfindungsgemäßen Vorrichtung (1) auch Verfahrensmerkmale aufgeführt werden, beziehen sich diese vorzugsweise auf das erfindungsgemäße Verfahren, das nachstehend noch näher definiert wird. Ebenso beziehen sich Vorrichtungsmerkmale, die im Zusammenhang mit dem erfindungsgemäßen Verfahren genannt werden, vorzugsweise auf die erfindungsgemäße Vorrichtung (1).

Nachfolgend wird die Erfindung detaillierter beschrieben.

Ein erster Gegenstand der vorliegenden Erfindung ist eine Vorrichtung (1) zur Abtrennung eines Zielprodukts aus einer das Zielprodukt enthaltenden flüssigen Phase P. Die Vorrichtung (1) umfasst zumindest einen Primärraum (3) für ein Wärmeträgermedium W und zumindest eine erste Zufuhreinrichtung (5a) und eine erste Abfuhreinrichtung (5b) für das Wärmeträgermedium W. Ferner weist die Vorrichtung (1) zumindest einen Sekundärraum (7) für die flüssige Phase P und zumindest eine zweite Zufuhreinrichtung (9) für die flüssige Phase P auf. Der Primärraum (3) und der Sekundärraum (7) werden durch zumindest eine Kristallisationsfläche (13) getrennt. Zudem umfasst die Vorrichtung (1) zumindest eine zweite Abfuhreinrichtung (15) für das Zielprodukt. Dabei weist die Vorrichtung (1) ferner zumindest eine Aufgabeeinheit (11) für die flüssige Phase P im Wesentlichen direkt auf die Kristallisationsfläche (13) und/oder die Oberflächen von das Wärmeträgermedium W führenden Leitungen auf.

Wie vorstehend geschildert, können sich auf den Kristallisationsflächen (13) einer gattungsgemäßen Vorrichtung Kristallisatschichten und Verkrustungen des Zielprodukts bilden. Um die Kristallisatschichten und Verkrustungen effektiv und schonend entfernen zu können, umfasst die erfindungsgemäße Vorrichtung zumindest eine Aufgabeeinheit (11), mittels derer die flüssige Phase P im zumindest angewärmten Zustand im Wesentlichen direkt auf die Kristallisationsfläche (13) und/oder die Oberflächen von das Wärmeträgermedium W führenden Leitungen aufgegeben werden kann. Hierdurch können entstehende Kristallisatschichten und Verkrustungen gelöst, bzw. aufgetaut werden, ohne dass sich größere Stücke an Kristallisat lösen, bzw. wenn diese sich bilden, diese zu keine Schäden an während des Aufschmelzvorgangs mechanisch bewegten Teile führen.

In einer Weiterbildung der erfindungsgemäßen Vorrichtung (1) umfasst die Aufgabeeinheit (11) ein Rohrleitungssystem mit Düsen zum Verteilen der flüssigen Phase P₀ auf die mit Kristallisatschichten bzw. Verkrustungen belegten Kristallisationsfläche (13) und/oder die Oberflächen von das Wärmeträgermedium W führenden Leitungen. Vorteilhaft ist wenn die zugeführte flüssige Phase P vor der Verteilung auf eine Temperatur oberhalb der Kristallisations- bzw. Sättigungstemperatur erwärmt wird. Durch diese Ausführungen der Aufgabeeinheit (11) wird insbesondere sichergestellt, dass die flüssige Phase P gleichmäßig verteilt auf die jeweilige Kristallisationsfläche (13) und/oder Oberflächen von das Wärmeträgermedium W führenden Leitungen aufgebracht wird und damit gleichmäßig löst, bzw. abtaut.

In einer Ausführungsform der Erfindung kann die flüssige Phase P₀ die flüssige Phase P oder eine flüssige Phase P₁ umfassend zumindest ein Lösungsmittel aufweisen. Das Lösungsmittel ist bevorzugt Wasser.

Um das Abschmelzen, bzw. Ablösen der Kristallisatschichten oder Verkrustungen ohne Verunreinigung der flüssigen Phase P durchführen zu können, wird die Aufgabeeinheit vorzugsweise mit der flüssigen Phase P beaufschlagt, wobei über einen vorgeschalteten ersten Wärmetauscher (23) zugeführte flüssige Phase P auf eine Temperatur über dem Schmelzpunkt der Kristallisatschichten oder Verkrustungen eingestellt wird. Wird dabei die flüssige Phase P zur Aufgabeeinheit (11) beispielsweise über eine Pumpe (25) aus dem Sekundärraum (7) entnommen, erfolgt durch die damit erzeugte Umwälzung ein sehr effektives Abschmelzen, bzw. Ablösen der Kristallisatschichten oder Verkrustungen.

Es hat sich für ein schnelleres Abschmelzen, bzw. Ablösen der Kristallisatschichten oder Verkrustungen zudem als vorteilhaft herausgestellt, wenn die Vorrichtung (1) ferner einen ersten Wärmetauscher (19) für das Wärmeträgermedium W umfasst, der vor der ersten Zufuhreinrichtung (5a) angeordnet ist. Somit kann das Wärmeträgermedium W, das durch den jeweiligen Primärraum (3) strömt, vorgewärmt werden.

Besonders bevorzugt ist die Vorrichtung (1) als Kühlscheibenkristallisator ausgestaltet.

Ein zweiter Gegenstand der vorliegenden Erfindung ist ein Verfahren zur Abtrennung eines Zielprodukts aus einer das Zielprodukt enthaltenden flüssigen Phase P. Dieses Verfahren kann insbesondere mittels einer erfindungsgemäßen Vorrichtung (1), wie sie vorstehend beschrieben wurde, ausgeführt werden.

In einem Verfahrensschritt a) wird ein gekühltes Wärmeträgermedium W zu einem Primärraum (3) über eine erste Zufuhreinrichtung (5a) zugeführt. In einem Verfahrensschritt b) wird dann die flüssigen Phase P in einem vorgekühlten Zustand zu einem Sekundärraum (7) über eine zweite Zufuhreinrichtung (9) zugeführt, wobei durch einen Wärmeübergang aus der flüssigen Phase P durch eine zwischen dem Primärraum (3) und dem Sekundärraum (7) angeordnete Kristallisationsfläche (13) hindurch auf das Wärmeträgermedium W die Sättigungsgrenze des Zielprodukts in der flüssigen Phase P überschritten und dadurch das Zielprodukt an der Kristallisationsfläche (13) als Kristallisat A abgeschieden wird.

In einem Verfahrensschritt c) wird das Kristallisat A von der Kristallisationsfläche (13) entfernt und aus dem Sekundärraum (7) über eine zweite Abfuhreinrichtung (15) abgeführt. Gleichzeitig oder zeitlich versetzt wird in Schritt d) das erwärmte Wärmeträgermedium W aus dem Primärraum (3) über eine erste Abfuhreinrichtung (5b) abgeführt.

In vorbestimmten Zeitabständen werden dann die im Wesentlichen kontinuierlich ausgeführten Verfahrensschritte a), b), c) und d) angehalten und es wird Verfahrensschritt e) ausgeführt, in dem schließlich eine warme flüssige Phase P₀ mittels einer Aufgabeeinheit (11) im Wesentlichen direkt auf die Kristallisationsfläche (13) und/oder die Oberflächen von das Wärmeträgermedium W führenden Leitungen aufgegeben wird.

Unter "warme flüssige Phase P₀" wird eine flüssige Phase verstanden, deren Temperatur über dem Schmelzpunkt der Kristallisatschichten oder Verkrustungen liegt.

Wie vorstehend zur Vorrichtung (1) schon ausgeführt wurde, ermöglicht die Aufgabe von warmer flüssiger Phase P mittels der erfindungsgemäßen Aufgabeeinheit (11), entstehende Kristallisatschichten oder Verkrustungen schonend und schnell abzubauen, ohne dass sich Stücke davon lösen und Teile der Vorrichtung (1) mechanisch beschädigen. Indem die warme flüssige Phase P im Wesentlichen direkt auf die Kristallisationsfläche (13) und/oder die Oberflächen von das Wärmeträgermedium W führenden Leitungen aufgegeben wird, wird diese gezielt auf die betreffenden Flächen und Oberflächen gerichtet, um den gewünschten erfindungsgemäßen Effekt des Abschmelzens, bzw. Ablösens von Kristallisatschichten oder Verkrustungen zu bewirken.

In einer Weiterbildung des erfindungsgemäßen Verfahrens werden parallel zu Verfahrensschritt e) zumindest zeitweise die Verfahrensschritte aa) Zuführen eines erwärmten Wärmeträgermediums W zu dem Primärraum (3) über die erste Zufuhreinrichtung (5a) und/oder dd) Abführen des abgekühlten Wärmeträgermediums W aus dem Primärraum (3) über eine erste Abfuhreinrichtung (5b) ausgeführt. Hierdurch wird das vom Sekundärraum (7) aus bewirkte Abschmelzen bzw. Ablösen von Kristallisatschichten oder Verkrustungen aus dem Primärraum (5) heraus (d.h. aus dem Inneren der Kühlscheibe (103) unterstützt. Das gleiche gilt für die Leitungen, in denen das Wärmeträgermediums W strömt.

Alternativ oder zusätzlich kann parallel zu Verfahrensschritt e) zumindest zeitweise Verfahrensschritt f) Umwälzen der flüssigen Phase P aus dem Sekundärraum (7) über einen zweiten Wärmetauscher (23) und Zuführen der erwärmten flüssigen Phase P zu der Aufgabeeinheit (11) ausgeführt werden. Auch diese Maßnahme dient der weiteren Unterstützung des Abschmelzens bzw. Ablösens von Kristallisatschichten oder Verkrustungen.

In einer bevorzugten Ausführungsform ist die flüssige Phase P ein Produktgemisch, das Acrylsäure enthält, vorzugsweise mit einer Konzentration > 80 Gew.-%, und das Kristallisat A ist reine Acrylsäure, vorzugsweise mit einem Anteil > 99 Gew.-%.

Weitere Ziele, Merkmale, Vorteile und Anwendungsmöglichkeiten ergeben sich aus der nachfolgenden Beschreibung von Ausführungsbeispielen der vorliegenden Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den Ansprüchen oder deren Rückbeziehung.

Es zeigen:
- Figur 1: eine schematische Darstellung eines Kühlscheibenkristallisators 1 nach einer Ausführungsform der vorliegenden Erfindung und
- Figur 2: eine schematische Querschnitts-Darstellung einer Kühlscheibe 103 gemäß einer Ausführungsform der vorliegenden Erfindung.

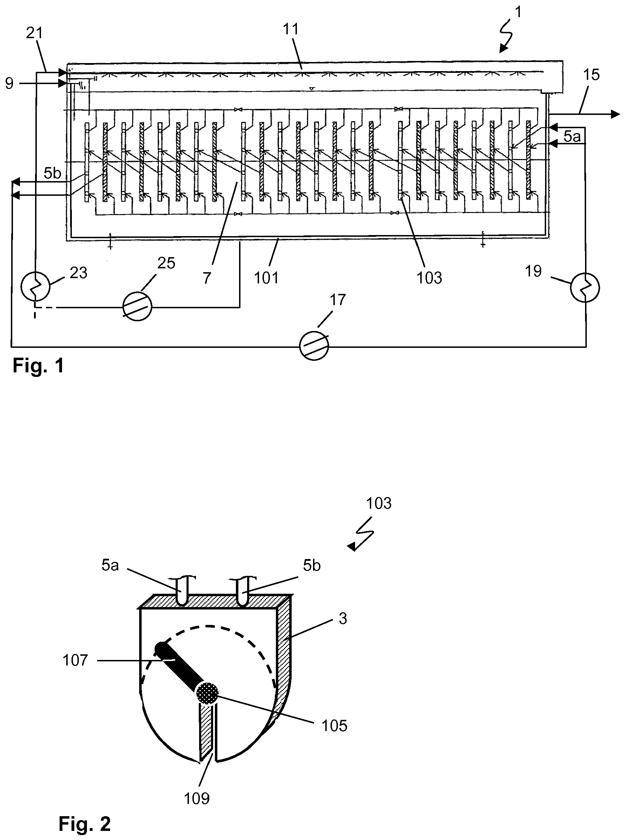

Figur 1 zeigt eine schematische Darstellung eines Kühlscheibenkristallisators 1 als eine bevorzugte Ausführungsform der erfindungsgemäßen Vorrichtung 1. Dabei handelt es sich um eine liegend angeordnete, gerührte Vorrichtung. Wie Figur 1 zu entnehmen ist, umfasst diese als Hauptbestandteile ein trogförmiges Gehäuse 101 und die im Gehäuse 101 senkrecht von oben eingehängten Kühlscheiben 103, die senkrecht zur Längsachse der Vorrichtung 1 und parallel hintereinander angeordnet sind. Durch das trogförmige Gehäuse 101 verläuft entlang der Längsachse eine horizontale Welle 105 mit radial angeordneten Wischern 107, was in Figur 1 nicht explizit dargestellt ist. Die Kühlscheiben 103 weisen in ihrer unteren Hälfte einen Ausschnitt 109 auf, um sie über die Welle 105 einschieben zu können (vgl. Figur 2).

Das Innere des trogförmigen Gehäuses 101 bildet im Wesentlichen den Sekundärraum 7 aus, in dem die flüssige Phase P aufgenommen ist, welche durch die zweite Zufuhreinrichtung 9 im Wesentlichen kontinuierlich zugeführt wird. Auf der gegenüberliegenden Seite der Vorrichtung 1 ist eine zweite Abfuhreinrichtung 15 für das Zielprodukt angeordnet. Hier wird im Wesentlichen eine Suspension S des kristallisierten Zielprodukts abgezogen. Ebenfalls kann eine dritte Abfuhreinrichtung 17 für Nebenprodukte N vorgesehen sein (hier nicht dargestellt).

Über die Kühlscheiben 103 erfolgt die indirekte Kühlung der zugeführten flüssigen Phase P. Die flüssige Phase P und das Wärmeträgermedium W, das in diesem Betriebsmodus als Kühlmittel wirkt, werden dabei im Gegenstrom durch die Vorrichtung 1 geführt. Die Kühlscheiben 103 bewirken eine weitgehende Unterteilung des Arbeitsvolumens, so dass eine nur schwache axiale Rückvermischung vorherrscht und sich entsprechende Temperaturen und (Feststoff)-Konzentrationsprofile einstellen können. Die mit der Welle 105 langsam rotierenden Wischer 107 sollen bewirken, dass die Kühlscheiben 103 weitgehend frei von Kristallisatschichten oder Aufwachsungen (sog. "fouling") bleiben und damit die kontinuierliche Wärmeabfuhr bzw. den kontinuierlichen Betrieb des Kühlscheibenkristallisators 1 ermöglichen. Wie vorstehend beschrieben, gibt es aber auf den Kühlscheiben 103 Bereiche, die von den Wischern 107 nicht erreicht werden, so dass sich dort Kristallisatschichten oder Aufwachsungen bilden können. Ebenso können sich diese Kristallisatschichten oder Aufwachsungen auf Leitungen bilden, durch welche das Wärmeträgermedium W in den Sekundärraum 7 hinein und zwischen den Kühlscheiben 103 durch diesen hindurch geleitet wird.

Oberhalb der Kühlscheiben 103 sind mehrere, im bevorzugten Fall für jede Kühlscheibe 103 eine oder mehrere Aufgabeeinheiten 11 angeordnet, durch welche flüssige Phase P auf die Kühlscheiben 103, insbesondere auf die Kristallisationsflächen 13, und die Oberflächen der das Wärmeträgermedium W führenden Leitungen aufgegeben wird. Die Aufgabeeinheiten 11 sind in der Regel als Rohrleitungssystem mit Düsen ausgeführt.

Im regulären Betrieb des Kühlscheibenkristallisators 1 wird durch die zweite Zufuhreinrichtung 9 vorgekühlte flüssige Phase P zugeführt, während kaltes oder gekühltes Wärmeträgermedium W im Gegenstrom durch die Kühlscheiben 103 geleitet wird, um eine optimale Abscheidung des Zielprodukts als Kristallisat zu erreichen. Dies wird nachstehend noch weiter ausgeführt.

Im Regenerationsbetrieb des Kühlscheibenkristallisators 1 wird hingegen die Zufuhr von vorgekühlter flüssiger Phase P angehalten, und stattdessen wird die Aufgabeeinheit 11 vorzugsweise mit vorgewärmter flüssiger Phase P₀ versorgt, um das Aufschmelzen, bzw. Abtauen von Kristallisatschichten oder Verkrustungen auf den Kristallisationsflächen 13 zu beschleunigen. Zum Erwärmen kann der zweite Wärmetauscher 23 dienen. Die hierzu verwendete flüssige Phase P₀ kann insbesondere in Form der flüssigen Phase P aus dem Sekundärraum 7 abgezogen werden, vorzugsweise über einen in den Figuren nicht dargestellten Ablauf, der sich bevorzugt im oberen Teil des Sekundärraumes 7 befindet, um eine möglichst feststofffreie flüssiger Phase P einer Zirkulationspumpe 25 zuzuführen.

In einer vereinfachten Ausführungsform kann die Aufgabeeinheit 11 aber auch mit der vorgekühlten flüssigen Phase P aus der zweiten Zufuhreinrichtung 9 versorgt werden, dann nimmt das Abschmelzen, bzw. Ablösen jedoch eine längere Zeit in Anspruch.

Zur weiteren Unterstützung wird zudem das Wärmeträgermedium W vom kalten Zustand in einen erwärmten Zustand gebracht, beispielsweise mit dem ersten Wärmetauscher 19, so dass das Abschmelzen, bzw. Ablösen durch Erwärmen der Kühlscheiben und Leitungen von innen beschleunigt wird.

Die in Figur 2 dargestellte Kühlscheibe 103 weist einen Primärraum 3 auf, durch welchen das Wärmeträgermedium W fließt, das über eine erste Zufuhreinrichtung 5a zugeführt und über eine erste Abfuhreinrichtung 5b abgeführt wird. Dieser Primärraum 3 wird hier im Wesentlichen von zwei Kristallisationsflächen 13 definiert und nach oben und unten in geeigneter Weise abgeschlossen. Die Kühlscheibe 103 weist ferner eine Aufnahme für eine Welle 105 auf, mit deren Drehbewegung langsam Wischer 107 rotiert werden.

Die einzelnen Primärräume 3 (in den einzelnen Kühlscheiben 103) sind untereinander verbunden, so dass das gekühlte Wärmeträgermedium W vorzugsweise im Gegenstrom zu der vorgekühlten flüssigen Phase P strömt. Dabei können die Kühlscheiben 103 in zwei parallele Züge aufgeteilt sein, um einen guten inneren Wärmeübergangskoeffizienten zu erhalten, d.h. eine ausreichend hohe Strömungsgeschwindigkeit des Wärmeträgermediums W durch die Primärräume 5 bei gleichzeitigem Begrenzen des summarischen Druckabfalls über die Kühlscheiben 103 sicherzustellen. Zudem wird eine gute Integrierbarkeit der Verrohrung in der Vorrichtung 1 ermöglicht.

In einer konkreten Ausführungsform kühlt durch die Übertragung von Wärme aus der dem Sekundärraum 7 zugeführten (und diesen in der Regel durchströmenden), Acrylsäure enthaltenden flüssigen Phase P durch die den Sekundärraum 7 und den wenigstens einen Primärraum 3 voneinander trennende Kristallisationsfläche 13 hindurch in ein in dem wenigstens einen Primärraum 3 fließendes kaltes oder gekühltes Wärmeträgermedium W hinein die flüssige Phase P ab, bis seine Sättigungsgrenze mit Acrylsäure überschritten ist und die flüssige Phase P der Übersättigung durch Ausbildung (d.h. Ausscheidung) von aus Acrylsäure aufgebautem Kristallisat A (als Zielprodukt) entgegenwirkt.

Ist der gewünschte Kristallisationsgrad erreicht (der Begriff Kristallisationsgrad meint dabei den Massenbruch oder auch Massenanteil des in der resultierenden Suspension von Kristallen der Acrylsäure in (flüssig) verbliebener Mutterlauge enthaltenen feinteiligen Kristallisats an der Gesamtmasse der Kristallsuspension), wird die Suspension S aus dem Sekundärraum 7 über die zweite Abfuhreinrichtung 15 herausgeführt. Durch Abtrennen der gebildeten Acrylsäurekristalls A von der sog. Mutterlauge kann die Acrylsäure in entsprechender Reinheit aus der Suspension S isoliert werden.

Der Begriff Mutterlauge wird dabei so verstanden, dass er sowohl Schmelzen (in ihnen entfällt ein Gewichtsanteil von ≥ 50 Gew.-% auf die Acrylsäure) aus Acrylsäure und Verunreinigungen, als auch Lösungen der Acrylsäure und diese gegebenenfalls begleitende Verunreinigungen in Lösungsmitteln oder in Lösungsmittelgemischen (auf die Acrylsäure entfällt in ihnen ein Gewichtsanteil von ≥ 50 Gew.-%) mit der Maßgabe umfasst, dass bei ihrer Abkühlung (d. h., bei der Abkühlung der Mutterlauge) die Acrylsäure auskristallisiert.

In einer Weiterbildung der Kühlscheibe 103, wie sie vorzugsweise in der Vorrichtung 1 aus Figur 1 eingebaut sein kann, besitzt diese Kühlscheibe 103 einen in zwei voneinander getrennte Strömungsräume aufgeteilten Primärraum 5. Der Haupt-Strömungsraum wird im regulären Betrieb von kaltem Wärmeträgermedium W durchströmt und ist mit einer Reihe von Leit- und Umlenkblechen versehen, um eine gute Strömungsführung zu erhalten, das heißt, einen guten Wärmeübergang bei geringem Druckverlust. Der Neben-Strömungsraum besteht aus einem um den Umfang des Haupt-Strömungsraums gelegten Rohr, das von warmem Wärmeträgermedium W durchströmt wird. Auf diese Weise lassen sich bis zu einem gewissen Grad Verkrustungen auf den ungewischten Flächen der Kühlscheibe 103 zumindest reduzieren. Kaltes und warmes Wärmeträgermedium W werden jeweils senkrecht von oben in die Kühlscheibe 103 hinein und aus ihr herausgeführt.

In einem großtechnisch ausgeführten Kühlscheibenkristallisator 1 nach einer Ausführung der Erfindung, sind 24 Kühlscheiben 103 vorgesehen. Die Hauptabmessungen des Kühlscheibenkristallisators 1 betragen 9,4 m Länge, 3,7 m Breite und ca. 4 m Höhe mit einem aktiven Suspensionsvolumen von etwa 76 m³. Mit den Tragvorrichtungen für die außenliegende Wellenlagerung und dem Antrieb beläuft sich die Gesamtlänge des Kühlscheibenkristallisators 1 auf etwa 12 m.

## Patentansprüche

1. Vorrichtung (1) zur Abtrennung eines Zielprodukts aus einer das Zielprodukt enthaltenden flüssigen Phase P, wobei die Vorrichtung (1) ein Kühlscheibenkristallisator ist, umfassend
- zumindest eine Kühlscheibe (103), die einen Primärraum (3) für ein Wärmeträgermedium aufweist,
- zumindest eine erste Zufuhreinrichtung (5a) und eine erste Abfuhreinrichtung (5b) für das Wärmeträgermedium W,
- zumindest einen Sekundärraum (7) für die flüssige Phase P,
- zumindest eine zweite Zufuhreinrichtung (9) für die flüssige Phase P,
- zumindest eine den Primärraum (3) und den Sekundärraum (7) trennende Kristallisationsfläche (13) und
- zumindest eine zweite Abfuhreinrichtung (15) für das Zielprodukt, **dadurch gekennzeichnet dass** die Vorrichtung
- zumindest eine Aufgabeeinheit (11) für eine flüssige Phase P₀ direkt auf die Kristallisationsfläche (13) und/oder die Oberflächen von das Wärmeträgermedium W führenden Leitungen aufweist,
wobei die Aufgabeeinheit (11) ein Rohrleitungssystem mit Düsen zum Verteilen der flüssigen Phase P₀ auf die Kristallisationsfläche (13) und/oder die Oberflächen von das Wärmeträgermedium W führenden Leitungen umfasst.

2. Vorrichtung (1) nach Anspruch 1, wobei die flüssige Phase P₀ die flüssige Phase P oder eine flüssige Phase P₁ umfassend zumindest ein Lösungsmittel aufweist.

3. Vorrichtung (1) nach einem der Ansprüche 1 oder 2, ferner umfassend einen ersten Wärmetauscher (19) für das Wärmeträgermedium W, der vor der ersten Zufuhreinrichtung (5a) angeordnet ist.

4. Verfahren zur Abtrennung eines Zielprodukts aus einer das Zielprodukt enthaltenden flüssigen Phase P, umfassend die Verfahrensschritte:
a) Zuführen eines gekühlten Wärmeträgermediums W zu einem Primärraum (3) einer Kühlscheibe (103) über eine erste Zufuhreinrichtung (5a),
b) Zuführen der flüssigen Phase P in einem vorgekühlten Zustand zu einem Sekundärraum (7) über eine zweite Zufuhreinrichtung (9), wobei durch einen Wärmeübergang aus der flüssigen Phase P durch eine zwischen dem Primärraum (3) und dem Sekundärraum (7) angeordnete Kristallisationsfläche (13) hindurch auf das Wärmeträgermedium W die Sättigungsgrenze des Zielprodukts in der flüssigen Phase P überschritten und dadurch das Zielprodukt an der Kristallisationsfläche (13) als Kristallisat A abgeschieden wird,
c) Entfernen des Kristallisats A von der Kristallisationsfläche (13) und Abführen aus dem Sekundärraum (7) über eine zweite Abfuhreinrichtung (15) und
d) Abführen des erwärmten Wärmeträgermediums W aus dem Primärraum (3) über eine erste Abfuhreinrichtung (5b),
wobei in vorbestimmten Zeitabständen die im Wesentlichen kontinuierlich ausgeführten Verfahrensschritte a), b), c) und d) angehalten werden und Verfahrensschritt e) ausgeführt wird
e) Aufgeben einer warmen flüssigen Phase P₀, deren Temperatur über dem Schmelzpunkt der Kristallisatschichten oder Verkrustungen liegt, mittels einer Aufgabeeinheit (11) im Wesentlichen direkt auf die Kristallisationsfläche (13) und/oder die Oberflächen von das Wärmeträgermedium W führenden Leitungen,
wobei die Aufgabeeinheit (11) ein Rohrleitungssystem mit Düsen zum Verteilen der flüssigen Phase P₀ auf die Kristallisationsfläche (13) und/oder die Oberflächen von das Wärmeträgermedium W führenden Leitungen umfasst.

5. Verfahren nach Anspruch 4, wobei die flüssige Phase P₀ die flüssige Phase P oder eine flüssige Phase P₁ umfassend zumindest ein Lösungsmittel aufweist.

6. Verfahren nach Anspruch 4 oder 5, wobei parallel zu Verfahrensschritt e) zumindest zeitweise die Verfahrensschritte aa) und/oder dd) ausgeführt werden
aa) Zuführen eines erwärmten Wärmeträgermediums W zu dem Primärraum (3) über die erste Zufuhreinrichtung (5a),
dd) Abführen des abgekühlten Wärmeträgermediums W aus dem Primärraum (3) über eine erste Abfuhreinrichtung (5b).

7. Verfahren nach einem der Ansprüche 4 bis 6, wobei parallel zu Verfahrensschritt e) zumindest zeitweise Verfahrensschritt f) ausgeführt wird,
f) Umwälzen der flüssigen Phase P aus dem Sekundärraum (7) über einen zweiten Wärmetauscher (23) und Zuführen der erwärmten flüssigen Phase P zu der Aufgabeeinheit (11).

8. Verfahren nach einem der Ansprüche 4 bis 7, wobei die flüssigen Phase P ein Produktgemisch ist, das Acrylsäure enthält, und das Kristallisat A reine Acrylsäure ist.

## Claims

1. An apparatus (1) for separation of a target product from a liquid phase P comprising the target product, where the apparatus (1) is a cooling disk crystallizer, comprising
- at least one cooling disk (103) which has a primary space (3) for a heat transfer medium,
- at least one first feed unit (5a) and one first removal unit (5b) for the heat transfer medium W,
- at least one secondary space (7) for the liquid phase P,
- at least one second feed unit (9) for the liquid phase P,
- at least one crystallization surface (13) which divides the primary space (3) and the secondary space (7), and
- at least one second removal unit (15) for the target product, wherein the apparatus has at least one application unit (11) for a liquid phase P₀ directly to the crystallization surface (13) and/or the surfaces of lines that conduct the heat transfer medium W, where the application unit (11) comprises a pipeline system having nozzles for distributing the liquid phase P₀ over the crystallization surface (13) and/or the surfaces of lines that conduct the heat transfer medium W.

2. The apparatus (1) according to claim 1, wherein the liquid phase P₀ includes the liquid phase P or a liquid phase P₁ comprising at least one solvent.

3. The apparatus (1) according to either of claims 1 and 2, further comprising a first heat exchanger (19) for the heat transfer medium W, arranged upstream of the first feed unit (5a).

4. A process for separating a target product from a liquid phase P comprising the target product, comprising the process steps of:
a) feeding a cooled heat transfer medium W to a primary space (3) of a cooling disk (103) by means of a first feed unit (5a),
b) feeding the liquid phase P in a precooled state to a secondary space (7) by means of a second feed unit (9), in the course of which the saturation limit of the target product in the liquid phase P is exceeded as a result of heat transfer from the liquid phase P to the heat transfer medium W through a crystallization surface (13) disposed between the primary space (3) and the secondary space (7), resulting in deposition of the target product at the crystallization surface (13) as crystals A,
c) removing the crystals A from the crystallization surface (13) and removing them from the secondary space (7) by means of a second removal unit (15) and
d) removing the heated heat transfer medium W from the primary space (3) by means of a first removal unit (5b),
with stoppage of the essentially continuous process steps a), b), c) and d) at predetermined time intervals and execution of process step e) e) applying a warm liquid phase P₀, the temperature of which is above the melting point of the crystal layers or encrustations, by means of an application unit (11) essentially directly to the crystallization surface (13) and/or the surfaces of lines conducting the heat transfer medium W, where the application unit (11) comprises a pipeline system having nozzles for distributing the liquid phase P₀ over the crystallization surface (13) and/or the surfaces of lines that conduct the heat transfer medium W.

5. The process according to claim 4, wherein the liquid phase P₀ includes the liquid phase P or a liquid phase P₁ comprising at least one solvent.

6. The process according to claim 4 or 5, wherein process steps aa) and/or dd) are executed at least temporarily in parallel to process step e)
aa) feeding a heated heat transfer medium W to the primary space (3) by means of the first feed unit (5a) ,
dd) removing the cooled heat transfer medium W from the primary space (3) by means of a first removal unit (5b).

7. The process according to any of claims 4 to 6, wherein process step f) is executed at least temporarily in parallel to process step e)
f) circulating the liquid phase P from the secondary space (7) by means of a second heat exchanger (23) and feeding the heated liquid phase P to the application unit (11).

8. The process according to any of claims 4 to 7, wherein the liquid phase P is a product mixture comprising acrylic acid, and the crystals A are pure acrylic acid.

## Revendications

1. Dispositif (1) pour la séparation d'un produit cible d'une phase liquide P contenant le produit cible, le dispositif (1) étant un cristallisateur à disques de refroidissement, comprenant
- au moins un disque de refroidissement (103), qui présente un espace primaire (3) pour un milieu de liquide caloporteur,
- au moins un premier dispositif d'amenée (5a) et un premier dispositif d'évacuation (5b) pour le milieu de liquide caloporteur W,
- au moins un espace secondaire (7) pour la phase liquide P,
- au moins un deuxième dispositif d'amenée (9) pour la phase liquide P,
- au moins une surface de cristallisation (13) séparant l'espace primaire (3) et l'espace secondaire (7) et
- au moins un deuxième dispositif d'évacuation (15) pour le produit cible, **caractérisé en ce que** le dispositif présente au moins une unité de chargement (11) pour une phase liquide P₀ directement sur la surface de cristallisation (13) et/ou les surfaces des conduites conduisant le milieu de liquide caloporteur W,
l'unité de chargement (11) comprenant un système de tuyauterie muni de buses destinées à la répartition de la phase liquide P₀ sur la surface de cristallisation (13) et/ou sur les surfaces des conduites conduisant le milieu de liquide caloporteur W.

2. Dispositif (1) selon la revendication 1, dans lequel la phase liquide P₀, la phase liquide P ou une phase liquide P₁ comprend au moins un solvant.

3. Dispositif (1) selon l'une des revendications 1 et 2, comprenant en outre un premier échangeur de chaleur (19) pour le milieu de liquide caloporteur W, qui est disposé avant le premier dispositif d'amenée (5a).

4. Procédé pour la séparation d'un produit cible d'une phase liquide P contenant le produit cible, comprenant les étapes de procédé :
a) amenée d'un milieu de liquide caloporteur W refroidi à un espace primaire (3) d'un disque de refroidissement (103) par l'intermédiaire d'un premier dispositif d'amenée (5a),
b) amenée de la phase liquide P dans un état pré-refroidi à un espace secondaire (7) par l'intermédiaire d'un deuxième dispositif d'amenée (9), dans lequel, par un transfert de chaleur de la phase liquide P au milieu de liquide caloporteur W, à travers une surface de cristallisation (13) disposée entre l'espace primaire (3) et l'espace secondaire (7), la limite de saturation du produit cible dans la phase liquide P est dépassée et le produit cible est ainsi déposé en tant que cristallisat A sur la surface de cristallisation (13),
c) élimination du cristallisat A de la surface de cristallisation (13) et évacuation de l'espace secondaire (7) par l'intermédiaire d'un deuxième dispositif d'évacuation (15) et
d) évacuation du milieu de liquide caloporteur W réchauffé de l'espace primaire (3) par l'intermédiaire d'un deuxième dispositif d'évacuation (5b),
dans lequel, à des intervalles de temps prédéfinis, les étapes de procédé mises en œuvre essentiellement en continu a), b), c) et d) sont stoppées et l'étape de procédé e) est mise en œuvre
e) chargement d'une phase liquide chaude P₀, dont la température est supérieure au point de fusion des couches de cristallisat ou des incrustations, au moyen d'une unité de chargement (11), essentiellement directement sur la surface de cristallisation (13) et/ou les surfaces des conduites conduisant le milieu de liquide caloporteur W,
dans lequel l'unité de chargement (11) comprend un système de tuyauterie muni de buses destinées à la répartition de la phase liquide P₀ sur la surface de cristallisation (13) et/ou sur les surfaces des conduites conduisant le milieu de liquide caloporteur W.

5. Procédé selon la revendication 4, dans lequel la phase liquide P₀, la phase liquide P ou une phase liquide P₁ comprend au moins un solvant.

6. Procédé selon la revendication 4 ou 5, dans lequel en parallèle à l'étape de procédé e) les étapes de procédé aa) et/ou dd) sont mises en œuvre au moins temporairement
aa) amenée d'un milieu de liquide caloporteur W réchauffé à l'espace primaire (3) par l'intermédiaire d'un premier dispositif d'évacuation (5a),
dd) évacuation du milieu de liquide caloporteur W refroidi de l'espace primaire (3) par l'intermédiaire d'un premier dispositif d'évacuation (5b).

7. Procédé selon l'une quelconque des revendications 4 à 6, dans lequel en parallèle à l'étape de procédé e), l'étape de procédé f) est mise en œuvre temporairement,
f) recirculation de la phase liquide P de l'espace secondaire (7) par l'intermédiaire d'un deuxième échangeur de chaleur (23) et amenée de la phase liquide P réchauffée à l'unité de chargement (11).

8. Procédé selon l'une quelconque des revendications 4 à 7, dans lequel la phase liquide P est un mélange de produits qui contient de l'acide acrylique, et le cristallisat A est de l'acide acrylique pur.
